# EUROPEAN PATENT APPLICATION

(11) **EP 2 177 233 A1**
(43) Date of publication of application: **21.04.2010**
(21) Application number: 10151048.5
(22) Date of filing: 05.06.2003
(51) Int. Cl.: A61L 2/00, A61L 11/00, C02F 3/00

(54) **Methods of using odor control compositions**

(62) Divisional of application: 03731572.8
(71) Applicant: Biomagic, Inc., Costa Mesa CA 92627 (US)
(72) Inventor: Alfrey, Janice, Newport Beach, CA 92659-0188 (US); Alfrey, Paul, Newport Beach, CA 92659-0188 (US)
(74) Representative: Rupp, Christian

(57) **Abstract**

Odor control compositions include sources of nitrogen, a source of oxygen, and a surfactant. The compositions added to a biomass in a concentration sufficient to produce a noticeable stimulation of aerobic activity. Preferred compositions include at least one of ammonium nitrate and urea, as well as calcium nitrate, and more preferred compositions also include potassium nitrate. Related methods include use of the compositions in treating a wide range of odors emanating from sewage and other flowable materials.

## Description

### Background

Society is constantly trying to combat offensive odors. The sources of such odors are legion, with offensive odors emanating from areas where waste is stored, from laboratories or other commercial areas, combustion processes, from seafood industries or other processing areas, from farms or other areas where animals are housed, and from rest rooms and numerous other public areas. Offensive odors are also routinely generated by individual human activities, such as smoking and using cleaning products.

Various methods of combating odors are known. The simplest methods involve using fragrances or other strong smells to disguise or cover up offensive smells. It is also known to use compounds such as baking soda and activated charcoal that neutralize odors by absorption.. Unfortunately, the effect in such cases is only temporary reduction of odors. In addition, none of the above mentioned methods are effective enough against very strong or lingering odors, such as those that emanate from sewage or litter.

Another approach is to employ chemical or biological means to alter the composition of an odor. For example, foul smelling compounds can sometimes be chemically reduced or oxidized to a form that is not offensive. It is also known to employ bactericidal agents to kill or inactivate bacteria that are causing the bad odor. Still further, it is known to use enzymes to metabolize odorous compounds into non-odorous compounds. Unfortunately, even these strategies are not always effective, or at least not within cost effective parameters.

The effectiveness of bacterial decomposition of wastes depends in part on the extent to which the growth of the bacteria can be promoted. For example, waste treatment plants often use either aerobic or anaerobic digesters to reduce sludge, and otherwise prepare the waste for return to the environment. The aerobic digesters require a source of oxygen, which can be added as bubbled air, or other aeration or mixing technique. The bacterial action within the digesters can also be facilitated by addition of growth factors.

US 4911843 to Hunniford et al. (May 1990), reissued as RE36651 (Apr. 2000) and RE37181 (May 2001), teaches that that the addition of nitrate, via an aqueous sodium nitrate solution, to sewage systems, waste treatment plants and other industrial waste applications containing dissolved hydrogen sulfide, can be effective in eliminating or substantially reducing the hydrogen sulfide, as well as eliminating other "minor" odors associated with other sulfur-containing compounds. The theory is that the addition of nitrate provides an oxygen source which promotes the growth of naturally occurring bacteria which utilize in their metabolism the sulfur tied up as hydrogen sulfide. The Hunniford approach has been commercially successful in a product known as Bioxide™, which is sold by U.S. Filter Corporation for addition to digesters, cesspools, and the like.

The Bioxide™ formulation is mostly calcium nitrate. While the product is unquestionably effective in reducing hydrogen sulfide production from many wastes, it does not work as fast as one might like, and in some instances it must be present in fairly high concentrations to be effective. Thus, there is still a need for improved compounds and methods for reducing odors.

### Summary

The present invention provides compositions that include a source of nitrogen, a non-salt source of oxygen, and a surfactant, as well as various methods for producing, marketing, and using such compositions for treating odors and providing biostimulation of bacteria in a biomass.

Preferred compositions include at least one of ammonium nitrate and urea, as well as calcium nitrate. More preferred compositions also include potassium nitrate and/or a surfactant.

A preferred method of producing the odor control composition comprises combining a source of nitrogen, a non-salt source of oxygen, and a surfactant all in an aqueous solution. The nitrogen source is preferably a nitrate or urea, and the oxygen source is preferably a loosely associated oxygen. The odor control composition comprises at least 0.001 wt% of a nonyl-phenyl-ethoxylated compound or any other common surfactant.

The methods described herein treat various odors including sewage odors, odors emanating from an object, cat litter odors, and even room or air odors by applying an amount of the odor control composition to the odor and/or source of the odor. Those odors may be treated using various methods including spraying the surface of an object or the air with a mist, or even a dry fog, containing the odor control composition.

With reference to the methods of stimulating activity of bacteria, it is preferred that an amount of the odor control composition is added to the bacteria in a biomass in a concentration sufficient to produce a noticeable stimulation of aerobic activity.

Various objects, features, aspects and advantages of the present invention will become more apparent from the following detailed description of preferred embodiments of the invention.

### Detailed Description

The underlying theory is that the aerobic activity of bacteria in a biomass may be stimulated by adding bioavailable sources of nitrogen and oxygen. Growth of the bacteria in such an environment reduces undesirable emissions, including especially hydrogen sulfide. Obligate aerobes obviously will not change the nature of their metabolism, but facultative bacteria can be encouraged to switch to an aerobic mode in which they produce less odorous waste and decompose biomass. Of particular interest is the enteric group bacteria comprising the enterobacteracae family, denitrifying bacteria, and other types of facultative bacteria. Some contemplated aerobic bacteria include nitrifying bacteria, sulfur oxidizing bacteria, methane oxidizing bacteria, pseudomonas, etc. It is contemplated that enhanced aerobic activity may result in accelerated decomposition of organic matter in a biomass. For example, solids or sludge may be decreased in a biomass such as sewage.

### Nitrogen Source(s)

Many sources of nitrogen may be used, as long as the nitrogen is bioavailable. It is known, for example, that bacteria can metabolize nitrates, nitrites, amino acids, urea, uric acid, and creatinine. In preferred embodiments, the source of nitrogen is either nitrates or urea because those sources are relatively inexpensive, and are especially nutritious for bacteria. In terms of nitrates, all common and structurally stable nitrates may be used as long as they provide an available nitrogen source to facultative and/or aerobic bacteria. Some common examples of nitrates include HNO₃, NaNO₃, LiNO₃, KNO₃, RbNO₃, FrNO₃, Be(NO₃)₂, Mg(NO₃)₂, Ca(NO₃)₂, Sr(NO₃)₂, Ba(NO₃)₂, Ra(NO₃)₂, NH₄NO₃, and even Ag(NO₃)₂. Preferred nitrates include potassium nitrate, calcium nitrate, and ammonium nitrate because they provide further nutrition for facultative and/or aerobic bacteria.

Odor control compositions of the present invention may comprise only one nitrogen source, including for example, potassium nitrate, ammonium nitrate, or creatinine. However, it is also contemplated that a combination of various nitrogen sources may be advantageous, as different sources may be preferentially utilized by different bacteria. In most preferred embodiments, the odor control composition comprises calcium nitrate, ammonium nitrate, potassium nitrate, and urea.

Contrary to the teachings of the Hunniford et al. patents, we have found experimentally that the percentage of nitrogen in the composition is not especially critical. We have found compositions having at least 5% of the aqueous solution can be reasonably effective, and compositions having between 10 and 70 percent nitrogen to be even more effective. The amounts of nitrogen sources that are used depend on various factors including types of bacteria involved, temperature, types of odors, strength of the odor, amounts of bacteria present, type and composition of the environment (i.e. volume of water, sewage, air, etc.) to be treated, as well as various other factors that may also come into play. Our current preference is for compositions having between 2 and 50 percent nitrogen. When urea is used, we have found that bacteria grow well in compositions having approximately 2-40% urea, and more preferably between 15-30% urea. In terms of dry weight percents, we have found useful formulations to include at least 2% of at least one of ammonium nitrate and calcium nitrate, urea comprises at least 2%, and potassium nitrate comprises at least 0.01 %.

We have also found it to be advantageous to have a total amount of ammonium nitrate, calcium nitrate, and potassium nitrate that is present in an amount between 5-50 wt%. It is especially preferred that the composition comprise a total of 54 wt% of ammonium nitrate, calcium nitrate, and potassium nitrate. One class of especially preferred formulations includes 1-20 wt% calcium nitrate, especially 15 wt%, 2-40 wt% ammonium nitrate, especially 39 wt%, 0.01-12 wt% potassium nitrate, especially 0.1 wt%, and 2-30 wt% urea, especially 20 wt%. In another class of preferred formulations, the composition comprises 30-60 wt% ammonium nitrate, 10-30 wt% calcium nitrate, 1-10 wt% sodium nitrate, 5-10 wt% uric acid, and 1-10 wt% glycine. Another class of preferred formulations includes 10-40 wt% potassium nitrate and 2-20 wt% urea.

### Oxygen Source(s)

Preferred oxygen sources are those having loosely associated oxygen, defined herein to mean oxygen other than O₂ that facultative and aerobic bacteria can readily metabolize. Loosely associated oxygen can be covalently or ionically bound, and typically includes at least one hydrogen bond. Preferred sources of loosely associated oxygen are nitrates, sulfates, sulfites, phosphates, phosphates, and urea. Specific examples include H₂SO₄, Na₂SO₄, Li₂SO₄, K₂SO₄, Rb₂SO₄, Cs₂SO₄, Fr₂SO₄, BeSO₄, MgSO₄, CaSO₄, SrSO₄, BaSO₄, RaSO₄, H₂PO₄, Na₂PO₄, Li₂PO₄, K₂PO₄, Rb₂PO₄, Cs₂PO₄, Fr₂PO₄, BePO₄, MgPO₄, CaPO₄, SrPO₄, BaPO₄, RaPO₄. Other contemplated oxygen sources include carbohydrates, sugars, etc. In addition to providing bacteria with oxygen, carbohydrates and sugars also provide bacteria with energy.

The oxygen source(es) can be the same as the nitrogen source(s) discussed above, and such formulations are preferred. For example, in most preferred embodiments, the oxygen source is a loosely associated oxygen that is derived from a nitrate, urea, or combination of both. The oxygen can be present in any amount as long as it is not lethal to the bacterial population.

Non-salt forms of oxygen are preferred because they are relatively stable and are readily available for use or consumption by bacteria. Additionally, the non-salt forms of oxygen may be more readily consumed by bacteria and may promote a hypergrowth state in such bacteria.

### Surfactant(s)

While not wanting to be limited to any particular theory in this or any other aspect of the application, we currently think that a surfactant helps the bacteria feed on the nitrogen and oxygen sources. We also think that surfactants aid in the mixing of the nitrogen and oxygen source(s).

Practically any surfactant can be utilized, as used as long as it is mild and does not tend to disrupt the cellular membrane of microorganisms. Suitable surfactants include those commonly found in soaps, shampoos, detergents, as well as wetting agents. Although both ionic and non-ionic surfactants are contemplated, non-ionic surfactants are preferred because they tend not to alter the pH of the composition. Having tested numerous classes of surfactants, we now prefer nonyl-phenyl-ethoxylated surfactants. In preferred embodiments, 0.001-5 wt% surfactant is used, while in most preferred embodiments, only 0.02 wt% surfactant is used. Thus, one need not use a large amount of surfactant, but it is contemplated that more than one type of surfactant may be used to improve the effectiveness of the composition.

### Mixing

As claimed herein, it is contemplated to produce an odor control composition by combining in an aqueous solution a source of nitrogen, a non-salt source of oxygen, and a surfactant. That should be read as meaning that the combination is produced for the purpose of producing an odor control composition in the manner claimed.

Preferred odor control compositions and methods include water or other aqueous fluid in addition to the nitrogen source(s), oxygen source(s), and surfactant(s). The various ingredients should be thoroughly mixed, preferably via an automatic mixer but may also be accomplished manually, depending on the amount of composition being made. After mixing, the composition is typically pH adjusted to a neutral pH, using an acid (i.e. 6M HNO₃ or 20M HCl) and base (i.e. 6M NH₄OH or 16M NaOH). A non-aqueous fluid may be added to the composition, but such compositions are generally not preferred because they are often harder to work with.

Although various concentrations of the composition have been described herein, it may be advantageous to further dilute or concentrate the composition for use. For example, perhaps a concentrated solution is preferable for sale to keep shipping costs down and to minimize shelf space. Alternatively, perhaps a diluted composition may be preferable for sale so as to reduce any possible hazards in storage, handling, and transport of the composition.

Furthermore, the concentration of the composition may vary depending on several factors, including the amount of odor that needs to be controlled; the use of the composition; the strength and/or effectiveness of the composition; environmental conditions including temperature, humidity, etc.; amount of nitrogen and oxygen source(s) in the composition; the type and amounts and types of bacteria present in a biomass if applicable; and various other factors. Exemplary dilutions include 1:50, 1:100, and even 1:500 depending on various factors. In a preferred embodiment, a concentrated composition is sold, and the end user may dilute the product to 1:10, 1:100, 1:500, 1:10,000, or even 1:40,000, depending on the desired effectiveness of the composition. It is presently thought that in the treatment of airborne odors, the product would not be diluted to more than 1:700.

### Marketing And Usage

The preferred method of marketed is to package same with labeling identifying the composition as having efficacy in controlling odors, and offering the same for sale to businesses, households, governments, water districts, and so on. All manner of suitable packaging is contemplated including spray or non-spray bottles for individual use, as well as drum or other bulk packaging. Even small, personal size spray bottles are contemplated that can be readily carried on one's person or in one's luggage to treat odors in carpet or bedding of hotel rooms, and in bathrooms and other places subject to multiple person usage. The odor control compositions can be sold for various purposes, including deodorizing a room, area, or industry, and even for use as a plant growth stimulant.

The term "efficacy" or "effective in controlling odors" as used herein means a significant, noticeable reduction of odor recognizable to a human. It is preferable that the odor controlling compound be able to reduce the odor to a level that would be unnoticeable to humans, or below the "odor threshold," for a given substance. Standard odor thresholds for various substances are published and can be readily attained through various websites on the Internet. Additionally, the label may contain specific information pertaining to ingredients used in the product, directions for use of the product, safety information regarding use of the product, etc. The product can advantageously be offered for sale in a variety of ways, including especially through infomercials. A few examples include advertising the sale of the product through radio, television, newspapers or other periodicals, brochures; introducing the product in a trade show; and selling the product in a store or over the internet.

Odors lingering in the air can be treated, and often eliminated, by spraying a mist or fog of the odor control composition into the air. A manually operated spray bottle or an aerosol container can be used for purpose, although the mist or fog can also be supplied through a forced air heating or cooling system, or other automated delivery system. In waste plants, factories, and other areas subject to repeated odor problems, the spraying may advantageously be programmed to occur automatically at certain time periods throughout the day.

A fog containing the contemplated odor controlling solution can also be used. In this instance a fog is defined as a microvapor, having particles less than 25 microns in average diameter. To avoid possible lung problems, the fog particles should be safe for humans to breathe. Among other things, preferred fogs contain particles having an average size of at least 2 microns in size, more preferably at least 5 microns, and even more preferably at least 10 microns. The amount of fog used will depend upon numerous factors, including the amount of odor that needs to be controlled. In one waste processing facility, we have found that only 10 ounces of odor control composition per day is needed to adequately control odors in a 3600 square foot facility. Suitable fogs can be created using an ultrasonic vaporizer, which can advantageously be placed at the top of a room. The fog travels downward, eliminating odor in the air as it travels to the ground, and continues working after hitting the ground. Disbursement can also be accomplished manually, or through a forced air system as described above.

The above-mentioned methods of treating air with an odor controlling compound may be used in virtually any public or private place experiencing problems with odor, unless of course there is a contraindication. Particular applicability is seen for households, offices, factories and other commercial establishments, rest rooms and other public areas, sewage and trash treatment plants, farms and other agricultural areas, and medical facilities.

Odor control compositions can be used in the air (as discussed above), on solid surfaces, and also added to liquids and other fluids (such as that found in waste treatment plants). Typically, a solid surface of the substance is sprayed or rubbed with the odor controlling solution. This especially includes floors, toilets, beds, carpets, car seats, walls, trash cans, tools, clothes, kitty litter containers, etc.

Odor control compositions discussed herein can advantageously be added to sewage carried in a pipe, or settling in a vat, or present in any other treatment stage. For example, 1 ounce of a preferred composition can be sufficient, as an approximate starting dosage, in approximately 20 gallons of a liquid sewage-type waste in a holding tank to stimulate aerobic activity and to produce a reduction of the anaerobic activity, as measured by the amount of hydrogen sulfide remaining in the biomass. It is contemplated that a decrease in odor may be achieved in a matter of minutes. However, a decrease in odor may take longer depending on the conditions present in the system being treated, the amounts and types of bacteria present, various environmental factors, etc. For example, in a force-fed sewage pipe that carries approximately 300,000 gallons of sewage, initial odor control may be attained even in minutes when approximately 5 gallons of odor control composition is added on a daily basis, while further odor control (long-term odor control) may be attained as a uniform distribution is achieved.

Experimentally, we have found that odor control compositions can be cost-effectively added to sewage in sufficient quantity to produce a noticeable decrease in FOG (fats, oils, and grease) in pipes over a period of time via biostimulation of bacteria or a related mechanism. The amount of time needed to produce a noticeable decrease in FOG is dependent on multiple factors such as temperature, amount of FOG present, amount of odor to be eliminated, amount of composition used, amounts and types of facultative or aerobic bacteria present, and rate at which bacteria to respond to the composition. Thus, the period could vary anywhere from 5 days to 8 months, preferably at least 2 weeks, or even longer if the conditions are very unsuitable.

Bodies of water or other fluids may also be treated with the odor control composition. There, the odor controlling fluid could be added directly to the body of water or fluid to reduce odor. In particularly contemplated embodiments, preferred odor control compositions are sufficiently non-toxic that they can be safely added to water used for washing, rinsing, or treating equipment, floors, walls, solids, animal pens, various types of industries, etc.

Additionally, odor control compositions can also be used to biostimulate the bacteria that break down or digest waste products that occur in aqua culture or other similar or related processes.

Specific embodiments and applications of methods and compositions for odor control have been disclosed. It should be apparent, however, to those skilled in the art that many more modifications besides those already described are possible without departing from the inventive concepts herein. The inventive subject matter, therefore, is not to be restricted except in the spirit of the appended claims. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

## Claims

1. A method of stimulating aerobic activity of facultative bacteria in a biomass, comprising adding an amount of an odor control composition to the biomass in a concentration sufficient to produce a noticeable stimulation of the aerobic activity, wherein the odor control composition comprises:
a plurality of sources of nitrogen;
a source of oxygen selected from the group consisting of nitrates, sulfates, and phosphates, wherein the source of oxygen can be metabolized by facultative bacteria; and
a surfactant.

2. The method of claim 1, wherein producing a noticeable stimulation of the aerobic activity further comprises reducing odor to a level below an odor threshold for the biomass.

3. The method of claim 1, wherein the surfactant comprises at least 0.01 wt% of the odor control composition.

4. The method of claim 1, wherein the surfactant comprises at most 0.02 wt% of the odor control composition.

5. The method of claim 1, wherein the biomass comprises an amount of hydrogen sulfide.

6. The method of claim 5, wherein the biomass comprises bacteria that produce the amount of hydrogen sulfide.

7. The method of claim 1, wherein the biomass comprises enterobacteracae bacteria.

8. The method of claim 1, wherein the biomass comprises dentrifying bacteria.

9. The method of claim 1, wherein the noticeable stimulation of the aerobic activity reduces the volume of biomass.

10. The method of claim 1, wherein the stimulated aerobic activity reduces an amount of unwanted mass in the biomass, wherein the unwanted mass is selected from the group consisting of solids and sludge.

11. The method of claim 1, wherein the stimulated aerobic activity reduces an amount of unwanted mass in the biomass, wherein the unwanted mass is selected from the group consisting of fats, oils, and grease.

12. The method of claim 1, wherein the biomass comprises aerobic bacteria.
